# EUROPEAN PATENT APPLICATION

(11) **EP 1 312 611 A1**
(43) Date of publication of application: **21.05.2003**
(21) Application number: 00977971.1
(22) Date of filing: 28.11.2000
(51) Int. Cl.: C07K 1/12, C07C 229/02, C07C 227/18, A23J 3/30, A23L 1/305, G01N 33/68

(54) **METHOD OF CONTINUOUSLY DEGRADING PROTEIN**

(30) Priority: 21.08.2000 JP 2000250252
(71) Applicant: National Institute of Advanced Industrial Science and Technology, Tokyo 100-8901 (JP)
(72) Inventor: HATAKEDA, Kiyotaka, Sendai-shi, Miyagi 980-0874 (JP); IKUSHIMA, Yutaka, Sendai-shi, Miyagi 981-0962 (JP); MARUYAMA, Susumu, Tsukuba-shi, Ibaraki 305-0046 (JP); TORII, Kazuo, Sendai-shi, Miyagi 981-1105 (JP)
(74) Representative: Ebner von Eschenbach, Jennifer
(86) International application number: JP0008361
(87) International publication number: WO02016403

(57) **Abstract**

The present invention is aimed at providing a method for degrading protein into peptides and/or amino acids continuously and at a high rate in supercritical water or high-pressure hot water at near-supercritical point, and the method of the present invention related to a method for continuously hydrolyzing a substrate protein introduced into a reactor and manufacturing peptides and/or amino acids using a flow reactor which contains as constituent elements thereof a substrate introduction line for supplying a substrate, a preheating furnace for supplying high-temperature hot water, a reactor for heating and reacting a reaction solution, and a cooling unit for cooling the reaction solution, wherein the peptides and/or amino acids are manufactured from the protein continuously and at a high rate by means of continuous reaction in supercritical water or high-pressure hot water at near-supercritical point at a pressure of 22.05 MPa or greater and a temperature of 375°C or greater; and relates to a method for hydrolyzing protein by the abovementioned steps continuously and at a high rate, and is especially useful as a method for manufacturing physiologically active peptides and a preprocessing method or the like for evaluative analysis of amino acid compositions because of the ability to yield a preparation that has very few secondary and tertiary reaction products and is fundamentally different from a product obtained by a batch method.

## Description

### TECHNICAL FIELD

The present invention relates to a method of continuously hydrolyzing proteins to produce peptides and/or amino acids by continuous reaction using supercritical water or high-pressure hot water at near-supercritical point, and more particularly to a technique for manufacturing useful peptides and amino acids with fragments having near-natural amino acid sequences from ingredients containing protein, natural biological products, unused food products, or the like by continuous reaction under a supercritical state of the chemically stable and nonhazardous water, and to a preprocessing technique for analytically evaluating the amino acid composition thereof.

The present invention is useful as a continuous, high-speed production method of peptides and amino acids in which preparations that is unobtainable by a batch method and contaminated with very few secondary and tertiary reaction products can be obtained by the continuous reaction.

### BACKGROUND ART

Degradation of proteins is important as a means for knowing the structure and amino acid composition of proteins, and degradation of proteins has conventionally been performed by enzymatic cleavage and acid cleavage (references for enzymatic cleavage: New Course In Experimental Chemistry, Biochemistry (I) p. 156; references for acid cleavage: New Course In Experimental Chemistry, Biochemistry (I) pp. 156-157). A method based on high-temperature, high-pressure water to be performed by a batch process has also been proposed as a means for cleaving the peptide bonds of these proteins. Specifically, methods for hydrolyzing proteins to manufacture amino acids are disclosed in the references, which include a method for manufacturing amino acids from proteins characterized in performing hydrolysis thereof in water being in a supercritical or subcritical state, and a method for performing hydrolysis with a reaction time of 1-40 seconds at a temperature of 300-400°C and a pressure of 221 atm to 1000 atm (reference for batch method of thermolysis: Japanese Patent Application Laid-open No. H09-268166), but the methods in these reference consist of so-called batch methods, wherein the reaction solution is sealed in a container, which is introduced into a molten salt heated to between 180°C and 500°C, to raise the temperature thereof to critical temperature or higher. However, products by batch processes may contain products resulting from pathways of two, three, or more steps; and the amino acid sequence and the like of the resulting products differ completely from the amino acid sequences of the corresponding natural products; and the effects of the physiological activities thereof are effects that results from the complicated arrangement of the ingredients thereof.

Specifically, among these methods, the methods based on batch reactions performed at high temperature and high pressure have the following advantages and disadvantages:

One advantage lies in the fact that the form of the sample can be arbitrary. Disadvantages include the fact that batchwise reaction makes mass-production difficult, it is impossible to reliably evaluate the specified temperature because of the time required to reach this temperature, degradation with reaction times of under a few seconds cannot be performed, and the degradation products are likely to undergo secondary or tertiary reaction due to the inability to shorten the reaction time.

On the other hand, a method based on continuous reaction may have the following advantages and disadvantages:

Advantages include the fact that the reaction time can be extended to the range of 0.1 second or less, sharp analysis of the product is possible, this method is suitable for degradation and mass-production of low-molecular-weight peptides and amino acids, and the products can be applied to analytical evaluation of amino acids composed with few secondary or tertiary reaction.

Disadvantages include the inability to apply this method to proteins which cannot be introduced into the reaction vessel.

In the batch method of the prior art, 30-45 seconds are required for a reaction solution to reach the predetermined temperature of 400°C when the reaction is performed by using a reaction tube with a volume of approximately 10 mL under the conditions of 300 atm and 400°C, for example. Consequently, this method may not be applicable to degradation reactions with reaction times of 30-45 seconds or less. During a time period of 30-45 seconds for the retention time of the reaction solution which is a time for increasing the temperature thereof to 400°C, a material containing the corresponding products and secondary and tertiary reaction products of secondary and tertiary reactions is produced, and this material is unsuitable for analytical evaluation of the constitutional amino acids of the proteins.

### DISCLOSURE OF THE INVENTION

As a result of concentrated investigation conducted under these circumstances in view of the abovementioned prior art with the aim of making possible a degradation reaction with an extremely short reaction time falling well below 30-45 seconds in terms of the retention time of the reaction solution, and developing a new reaction method capable of continuously performing a degradation reaction in under a few seconds, during which no reaction products or secondary and tertiary reaction products would be contained therein, the inventors discovered that protein could be converted to peptides and/or amino acids continuously and at a high rate by performing continuous reaction in supercritical water using a specific flow reactor, and devised the present invention after further research.

An object of the present invention is to provide a method for manufacturing peptides and/or amino acids from protein by means of continuous reaction in supercritical water or high-pressure hot water at near-supercritical point.

Another object of the present invention is to provide a method for efficiently hydrolyzing protein into peptides . and/or amino acids continuously and at a high rate by continuous reaction in supercritical water or high-pressure hot water at near-supercritical point.

Further, another object of the present invention is to provide a production method of peptides and/or amino acids whereby a preparation having very few secondary and tertiary reaction products can be obtained and the products thus obtained are fragments that are closer to natural amino acid sequences in view of the fact that with the conventional batch method it is impossible to prevent situations in which products resulting from pathways of two, three, or more steps are contained, in which the amino acid sequence and the like of the product are completely different from the amino acid sequence of the corresponding natural product, and in which the activity thereof is an effect that results from the more complicated arrangement of the ingredients thereof.

The present invention, which is aimed at overcoming the abovementioned drawbacks, comprises the following technological means.
(1) A method for manufacturing peptides and/or amino acids from protein continuously wherein a substrate protein introduced into a reactor is hydrolyzed to peptides and/or amino acids by using a flow reactor which contains as constituent elements thereof a substrate introduction line for supplying a substrate, a preheating furnace for supplying high-temperature hot water, a reactor for heating and reacting a reaction solution, and a cooling unit for cooling the reaction solution, the method comprising reacting the substrate continuously in supercritical water or high-pressure hot water at near-supercritical point under a pressure of 22.05 MPa or greater and a temperature of 375°C or greater, and producing the peptides and/or amino acids from the protein continuously and at a high rate.
(2) A method for hydrolyzing proteins continuously wherein a substrate protein introduced into a reactor is converted to peptides and/or amino acids by using a flow reactor which contains as constituent elements thereof a substrate introduction line for supplying a substrate, a preheating furnace for supplying high-temperature hot water, a reactor for heating and reacting a reaction solution, and a cooling unit for cooling the reaction solution, the method comprises hydrolyzing the protein into peptides and/or amino acids continuously and at a high rate by using supercritical water or high-pressure hot water at near-supercritical point at a pressure of 22.05 MPa or greater and a temperature of 375° C or greater.
(3) The method according to the aforementioned (1) or (2), wherein the retention time of the reaction solution is from 0.001 second to 30 seconds.

### FORMS FOR ENFORCEMENT OF THE INVENTION

The present invention will next be described in further detail.

The degradation means by continuous reaction of the present invention comprises a method characterized in continuously degrading protein in a region of subcritical/supercritical water and obtaining a constituent peptide fragment or a constituent amino acid in the form of a fragment or a degraded preparation thereof having a near-natural amino acid sequence.

The apparatus for continuous reaction used in the present invention will be described based on the figures.

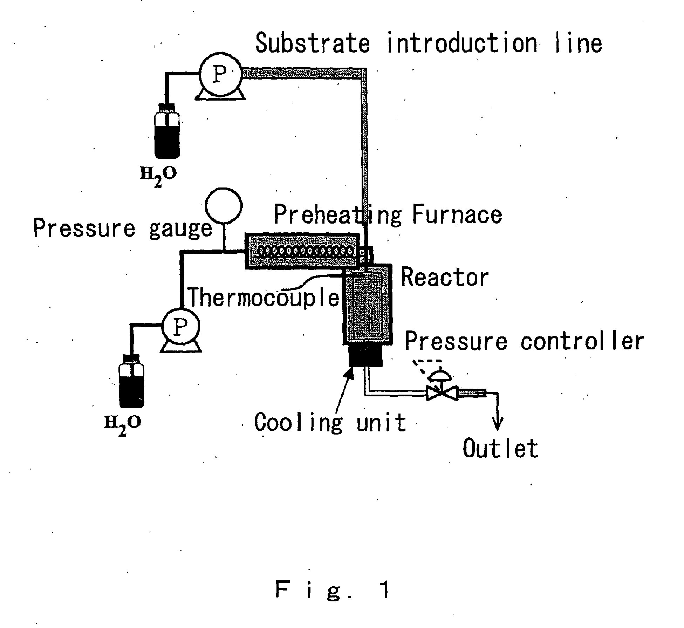

As an example of the apparatus for continuous reaction used in the present invention, as shown in Fig. 1, a apparatus for continuous reaction can be used, the apparatus comprising as constituent elements thereof a substrate introduction line for introducing a substrate using a high-pressure pump (pump used for high-speed liquid chromatography), a preheating furnace for preheating water sent from the high-pressure pump, a reactor for heating the reaction solution to a high-temperature hot water state and continuously reacting the substrate, a cooling unit for cooling the reaction solution, a pressure controller (pressure maintaining valve) for discharging the cooled reaction solution, a temperature gauge and pressure gauge (not shown).

In the abovementioned apparatus for continuous reaction, the substrate protein supplied from the substrate introduction line via, for example, a slurry filling machine is sent by the high-pressure pump, and is heated to about 400°C in the vicinity of the point of confluence of the preheating line and the substrate injecting line with high-temperature water heated in the preheating furnace, after which a specific quantity of substrate suspension is injected into the reactor and continuously reacted for a retention time of from 0.001 second to 1 second. The solution passed through the reactor is cooled to room temperature in the cooling unit, passed through the pressure controller (pressure maintaining valve), and recovered through an outlet.

The method of the present invention has operational effects whereby a technique extremely desirable in the chemical industry can be obtained as a result of the fact that protein can be continuously hydrolyzed into peptides and/or amino acids with the retention time of the reaction solution in the abovementioned reactor being from 0.001 second to 1 second, the recovery rate of peptides and/or amino acids resulting from amino acid analysis is extremely high in comparison with that by the batch method, these products can be manufactured continuously and at a high rate, the products obtained are fragments and degraded preparations that are closer to natural amino acid sequences, and other remarkable effects unattainable with the batch method can be obtained.

In the present invention, any protein, peptide, protein-containing ingredient, natural biological product, unused food product, resource, or other protein-containing type of protein material can be used as a substrate protein, and the type thereof is not particularly limited.

Water in a supercritical state at a pressure of 22.05 MPa or greater and a temperature of 375°C or greater can be given as a preferred example of supercritical water used in the hydrolysis reaction, but both high-pressure hot water at near-supercritical point and subcritical water can be used in the same manner even when either the pressure or temperature falls below these values, and the continuous reactions by that high-pressure hot water are also encompassed by the present invention.

The quantity of supercritical water used varies according to temperature and pressure conditions, supply quantity of carrier water, and quantity of introduced substrate, and is preferably 100-20,000 parts water per part protein in terms of weight, but the quantity used is not particularly limited.

The aforementioned flow reactor is preferably used as an apparatus for continuous reaction. For example, it is possible to use a device obtained by equipping the apparatus with a slurry filling machine for injecting a substrate to be hydrolyzed into the reaction vessel, a device equipped with an introducer or other unit having the same functions, or the like.

In the method of the present invention, degradation of a substrate protein is performed continuously in a few seconds, and the rate, temperature, pressure, and concentration for the degradation can be freely controlled by operating a valve. The resulting product can be used preferably for analytical evaluation of amino acid compositions because the product comprises fragments or degradation products that contain very few secondary and tertiary reaction products and are closer to natural amino acid sequences, which is fundamentally different from that by the batch reaction. Because of this, the method of the present invention can also be used preferably as a short-term preprocessing method for analytical evaluation of amino acid compositions.

Hydrolysis of protein by a batch reaction method using water in a subcritical state or supercritical state to hydrolyze protein is conventionally known, but the present invention comprises a continuous reaction method characterized in converting protein to peptides and/or amino acids continuously and at a high rate by continuous reaction in supercritical water using a flow reactor; and as a result of amino acid analysis of the products thus obtained, the method of the present invention allows the recovery rate of amino acids to be brought to a level that is considerably higher than in a batch method and that is close to the amino acid composition ratio provided by the hydrochloric acid hydrolysis of substrate protein, yields highly concentrated peptides unobtainable by the batch method, keeps the amount of byproducts at a low level, and provides other specific operational effects that cannot be expected from the batch method.

In a reaction according to the batch method, it takes 40-45 seconds for the protein to rise from room temperature to 400°C, and the protein is then exposed for 30 seconds under 400°C. At the same time, the pressure during this period is under conditions that vary from atmospheric pressure to 30 MPa.

Also, the retention time in the flow system is 0.006 second at 400°C and a constant pressure.

As a result of such reaction conditions, the composition of the former produced in the former case contains thermolysis products that range from room temperature to the critical temperature of water, and the thermolysis products undergo further hydrolysis and the like in a supercritical state, thus resulting in the possibility of products from pathways of two, three, or more steps being contained in the batch reaction. This potentially may cause the amino acid sequence or the like of the active substance to be totally different from that of the corresponding natural product. Constituent components thereof also are present in a wide variety. Consequently, the effects of the activity as such may result from a more complicated arrangement of the components thereof.

By contrast, the composition of the reaction material in the latter instance, which is based on an instantaneous reaction, can be considered from a completely different standpoint than the former. Specifically, the product thus obtained is a fragment or degraded product that is closer to a natural amino acid sequence. The activity of the component thus obtained is also sharper and is based on a more natural amino acid sequence, in contrast to the effects resulting from a complicated arrangement of components, as in the batch method; and the original effects of the active ingredients can be more clearly expressed. These are specific effects of the continuous reaction method which cannot be expected from a batch method, and are not only of utmost importance for the field of application of the active ingredients, but are also important for understanding negative effects.

### BRIEF DESCRIPTION OF THE DRAWINGS

Fig. 1 depicts an apparatus for continuous reaction (flow reactor) used in the present invention;
Fig. 2 depicts an apparatus for batch reaction; and
Fig. 3 depicts a comparison between the angiotensin-converting enzyme inhibition activity levels of flow reaction products and batch reaction products.

### BEST MODE FOR CARRYING OUT THE INVENTION

The present invention will next be described in detail based on examples, but the present invention is in no way limited by these examples.

### Example 1

Degradation by continuous reaction of zein (corn protein) substrate material in supercritical water with a reaction time of 0.7 second will be described in the present example.

Reaction conditions: an apparatus for continuous reaction is depicted in Fig. 1 for performing continuous reaction at 400° C and 300 atm.

An example is described below of protein hydrolysis by means of the flow reaction system of the present invention. Continuous reaction was performed using a flow reactor comprising a high-pressure pump (pump used for high-speed liquid chromatography), heating apparatus, reactor, cooling unit, pressure controller (pressure maintaining valve), temperature gauge, pressure gauge, and the like, in a reaction tube with a volume of 0.824 cubic centimeter (bore diameter 3.13 mm; length 11 cm, alloy C-276), with the reaction temperature and reaction pressure each being 400°C and 300 atm, respectively. The total amount of water sent from the high-pressure pump was 25 mL, comprising 20 mL from the preheating furnace and 5 mL from the substrate supply line. The internal temperature of the reaction tube was designed at this time to be 400°C. The reaction solution in this experiment had a retention time of 0.7 second. The retention time could also be freely controlled by the water supply rate. The pressure during progress of this experiment was within plus or minus 0.1 atm, and the temperature could be controlled to within plus or minus 0.2° C.

In these operations, a specific quantity of substrate suspension was injected after the temperature in the vicinity of the point of confluence of the preheating line and the substrate supply line was first increased to near 400°C. The solution that had passed through the reactor was cooled in the cooling unit, passed through the pressure maintaining valve, and recovered through an outlet. The results of amino acid analysis on the reaction material thus recovered are shown in column A of Table 1.

In the present example, the reaction solution was developed under the following conditions:
Substrate material used for degradation: zein (corn protein)
Substrate introduction method: continuous introduction
Substrate injection rate: 2 mg/min
Water supply rate: 25 mL/min
Volume of reaction vessel: 0.824 cm³
Retention time: 0.7 second

Column A in Table 1 consists of mole numbers and mole percentages for the results of amino acid analysis of the reaction solution in a continuous system. Column R in the table consists of 6 N hydrochloric acid hydrolysis values for zein. It is apparent that the continuous reaction of the present invention makes it possible to recover large amounts of amino acids, particularly those comprising glycine, alanine, proline, leucine, valine, phenylalanine and other proteins. It is also apparent that the amino acid recovery rate is significant, both qualitatively and quantitatively, in comparison with that by the batch method.

**TABLE 1**

| FREE AMINO ACID COMPOSITION OF CONTINUOUS AND BATCH REACTION PRODUCTS RESULTS OF AMINO ACID ANALYSIS | | | | | | |
|---|---|---|---|---|---|---|
| | CONTINUOUS SYSTEM | | | | | BATCH |
| | µM | | (MOL%) | | | µM |
| | A | B | A | B | R | |
| Asp | 3 | 170 | 0.1 | 6.5 | 5 | 11 |
| Thr | 4 | | 0.1 | | 2.9 | |
| Ser | 24 | 120 | 0.5 | 4.6 | 6.6 | |
| Glu | | 21 | 5 | 0.8 | 19.3 | 18 |
| Gly | 2600 | 360 | 57.3 | 13.8 | 2 | 48 |
| Ala | 830 | 580 | 18.3 | 22.2 | 13.7 | 85 |
| Cys | | | | | 0.4 | |
| Val | 140 | 89 | 3.1 | 3.4 | 3.7 | 22 |
| Met | 6 | 35 | 0.2 | 1.3 | 1.7 | |
| Ile | 39 | 110 | 0.9 | 4.2 | 3.7 | |
| Leu | 160 | 330 | 3.5 | 12.6 | 19.5 | 43 |
| Tyr | 28 | 100 | 0.6 | 3.8 | 3.6 | |
| Phe | 120 | 430 | 2.6 | 16.5 | 5.5 | 40 |
| Lys | 23 | 11 | 0.5 | 0.4 | 0.1 | |
| His | 27 | 85 | 0.6 | 3.3 | 1 | |
| Arg | 2 | 42 | 0.1 | 1.6 | 1.1 | |
| Pro | 530 | 130 | 11.7 | 5 | 10.1 | 5 |

### Example 2

Degradation of zein substrate material in supercritical water by continuous reaction with a reaction time of approximately 0.006 second will be described in the present example.

The volume of the reaction vessel was varied and continuous reaction was performed using the continuous reaction apparatus employed in example 1. A 1/16-inch (0.159-cm) reaction tube made of C-276 alloy and provided with a bore diameter of 0.2 mm and a length of 10 cm was used.

In the present example, the reaction conditions were 400° C and 300 atm, and a reaction solution was developed under the following conditions.
Substrate material used for degradation: zein (corn protein)
Substrate introduction method: continuous introduction
Substrate injection rate: 7.43 mg/min
Water supply rate: 17 mL/min
Volume of reaction vessel: 0.00314 cm³
Residence time: 0.006 second.

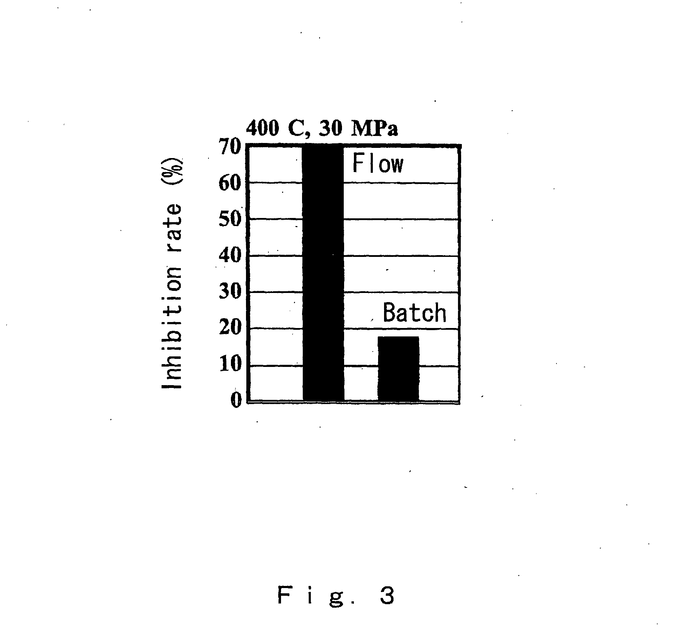

Column B in Table 1 consists of mole numbers and mole percentages for the results of amino acid analysis of the reaction solution in a continuous system. The continuous reaction of the present invention yields an amino acid composition ratio whose value approaches that of 6 N hydrochloric acid hydrolysis, except for glutamic acid, glycine, phenylalanine, and the like. It is possible that glutamic acid degraded and glycine was formed. A large quantity of peptide production is also apparent. Results of investigating the inhibitory activity of the product of the present invention with respect to the angiotensin-converting enzyme (there is an action that inhibits increases in blood pressure) show an inhibitory activity of 78%, and suitability thereof for the production of physiologically active substances is apparent. The results of investigating the angiotensin-converting enzyme inhibitory activity are shown in Fig. 3.

### Comparative Example

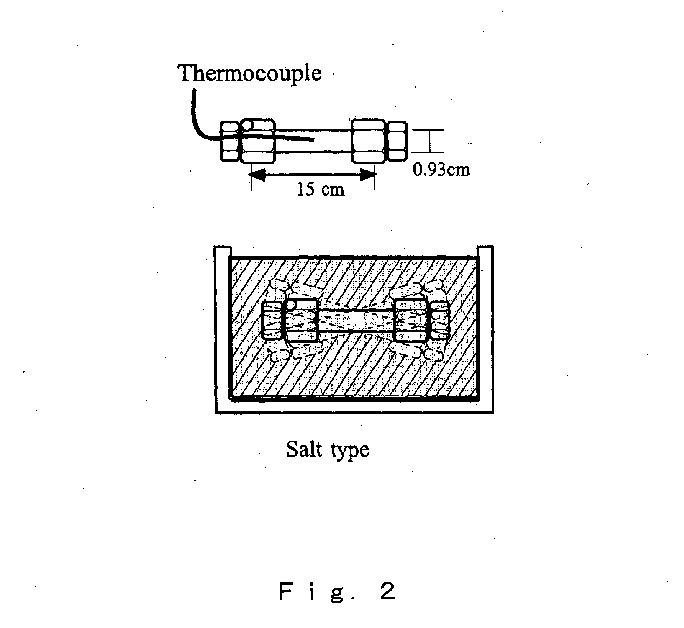

Hydrolysis of zein substrate material by the batch method will be described as the present comparative example. The batch reaction apparatus used in the present comparative example is depicted in Fig. 2. 100 mg of zein was placed in a stainless steel (SUS 316) container with a volume of 10.46 cm³, deoxygenated distilled water was sealed to bring about a watertightness of 358.05 (kg/m³) at 300 atm and 400° C, the interior of the tube was replaced with helium, the container was rendered airtight, and the product was introduced into molten salt (Salt type) at 400°C. The temperature inside the tube was measured by a thermocouple disposed in the vessel. The time required to reach the specified temperature of 400° C at that time was 45 seconds. After being held at 400°C for 30 seconds, the product was introduced into cooling water and the reaction was terminated. The results of amino acid analysis for this reaction solution are shown in the batch column of Table 1. The recovered amino acids consisted of 8 types, including alanine, guanine, and the like, and it is apparent that the types thereof were few and the recovered quantities were low in comparison with the product of the present invention. A low angiotensin-converting enzyme inhibition activity of 18% was also shown (Fig. 3).

### INDUSTRIAL APPLICABILITY

As heretofore described in detail, the present invention related to a method for continuously hydrolyzing a substrate protein introduced into a reactor and manufacturing peptides and/or amino acids using a flow reactor which comprises as constituent elements thereof a substrate introduction line for supplying a substrate, a preheating furnace for supplying high-temperature hot water, a reactor for heating and reacting a reaction solution, and a cooling unit for cooling the reaction solution; and relates to a method or the like for manufacturing peptides and/or amino acids from protein continuously and at a high rate by continuous reaction in supercritical water at a pressure of 22.05 MPa or greater and a temperature of 375°C or greater, and specific effects are achieved by means of the present invention whereby: 1) peptides and/or amino acids can be manufactured from protein continuously and at a high rate; 2) hydrolysis of protein can be continuously performed in a few seconds (retention time of the reaction solution may, for example, range from 0.001 second to 1 second); 3) the rate, temperature, pressure, and concentration for the degradation can be controlled in a continuous manner by operating a valve; 4) whereas a batch reaction allows products that result from pathways of two, three, or more steps to be contained, and the amino acid sequence or the like of the active substance to be completely different from the amino acid sequence of the corresponding natural product, the method of the present invention yields fragments or decomposition products that are closer to natural amino acid sequences, and produces preparations having very few secondary and tertiary reaction products; 5) a useful preprocessing method for evaluative analysis of amino acid structures can therefore be provided; 6) the proposed method is particularly useful as a manufacturing method for physiologically active peptides because the angiotensin-converting enzyme inhibition activity of the resulting product of the present invention is high in comparison with the batch method; and the like.

## Claims

1. A method for manufacturing peptides and/or amino acids from protein continuously wherein a substrate protein introduced into a reactor is hydrolyzed to peptides and/or amino acids by using a flow reactor which contains as constituent elements thereof a substrate introduction line for supplying a substrate, a preheating furnace for supplying high-temperature hot water, a reactor for heating and reacting a reaction solution, and a cooling unit for cooling the reaction solution, the method comprising reacting the substrate continuously in supercritical water or high-pressure hot water at near-supercritical point under a pressure of 22.05 MPa or greater and a temperature of 375°C or greater, and producing the peptides and/or amino acids from the protein continuously and at a high rate.

2. A method for hydrolyzing proteins continuously wherein a substrate protein introduced into a reactor is converted to peptides and/or amino acids by using a flow reactor which contains as constituent elements thereof a substrate introduction line for supplying a substrate, a preheating furnace for supplying high-temperature hot water, a reactor for heating and reacting a reaction solution, and a cooling unit for cooling the reaction solution, the method comprises hydrolyzing the protein into peptides and/or amino acids continuously and at a high rate by using supercritical water or high-pressure hot water at near-supercritical point at a pressure of 22.05 MPa or greater and a temperature of 375°C or greater.

3. The method according to claim 1 or 2, wherein the retention time of the reaction solution is from 0.001 second to 30 seconds.
